# FASCICULE DE BREVET EUROPEEN

(11) **EP 0 579 791 B1**
(45) Date de publication et mention de la délivrance du brevet: **24.05.2000**
(21) Numéro de dépôt: 93900251.5
(22) Date de dépôt: 13.11.1992
(51) Int. Cl.: C12N 5/10, A61K 48/00

(54) **Utilisation d'une composition cellulaire pour le traitement des organismes humains ou animaux**
Verwendung einer zellulären Zusammensetzung zur Behandlung von menschlichen oder tierischen Organismen
Use of a cellular composition for the traitement of human or animal organisms

(30) Priorité: 15.11.1991 FR 9114119
(43) Date de publication de la demande: 26.01.1994
(73) Titulaire: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: ROTH, Claude, F-75016 Paris (FR); MIR, Lluis, F-91370 Verrières-le-Buisson (FR); KOURILSKY, Philippe, F-75001 Paris (FR)
(74) Mandataire: Phélip, Bruno
(86) Numéro de dépôt international: FR9201061
(87) Numéro de publication internationale: WO9310219

(56) Documents cités:
- WO-A-90/06997
- WO-A-92/05262
- GOLUMBEK: "Treatment of established renal cancer by tumor cells engineered to secrete Interleukin-4", SCIENCE, , -01. Novembre 1991, Vol. 254, no. , pages 713 à 716

## Description

La présente invention a pour objet l'utilisation d'une composition cellulaire pour le traitement des organismes humains ou animaux.

Il a été établi très récemment , par diverses équipes scientifiques que l'injection localisée dans des organismes, atteints par une tumeur, de cellules tumorales syngéniques produisant une interleukine permettait le rejet de ces tumeurs par l'organisme.

Ceci a été mis en évidence pour l'interleukine-2 par Bubenik et al. ( Immunology Letters, 19 , 279-282, 1988 ; Immunology Letters, 23 , 287-292, 1989 ) et confirmé notamment par Fearon et al. (Cell., 6O, 397-4O3, 1990 ) et par Ley et al.,(European Journal of Immunology 1991, 21 : 851-854; Res. Immunol., 1990, 141:855-863 ).

Les auteurs de ces articles mentionnent que le rejet s'accompagne d'une mémorisation de la réponse. L'animal est ainsi vacciné contre le développement ultérieur d'une tumeur d'un même type , même si celle-ci a été greffée sur un site différent.

Des cellules cancéreuses syngéniques produisant l'interleukine-4 ont aussi été testées avec des résultats identiques , comme le rapportent Golumbek (Science , 254 , 713 - 716 , 1991 ) et Tepper et al. (Cell , 57,5O3-512, 1989) ainsi que des cellules produisant le facteur de nécrose des tumeurs (TNF) comme le décrit Blankenstein et al. (J. Exp. Med., 173, 1O47-1O52, 1991).

Les systèmes décrits dans ces publications présentent néanmoins des inconvénients en vue d'une utilisation en thérapie humaine.

En effet, dans toutes ces publications , les cellules produisant les interleukines sont des cellules de l'individu ou d'un individu syngénique , qui ont été modifiées afin qu'elles expriment l'interleukine.

Dans le cas d'une thérapie humaine , l'inconvénient majeur de cette méthodologie réside dans le fait que les cellules exprimant l'interleukine et injectées à l'organisme risquent de continuer à se développer même après le rejet de la tumeur.

Un deuxième inconvénient des techniques décrites dans l'art antérieur réside dans le mode d'insertion de l'ADN codant pour les interleukines , qui est le plus souvent fondé sur l'emploi de vecteurs viraux et notamment rétroviraux.

Ces méthodes présentent une bonne efficacité de transmission de l'ADN mais, l'utilisation de virus et notamment de rétrovirus peut présenter de graves inconvénients dans le cadre d'une thérapie humaine.

Ces méthodes , en outre nécessitent souvent une sélection rigoureuse des transfectants sur une longue période de temps et de ce fait sont difficilement applicables sur une large échelle en thérapie humaine notamment.

Il n'existe donc pas à la connaissance des demandeurs dans l'art antérieur de techniques fiables, faciles à mettre en oeuvre , et compatibles avec les impératifs de santé humaine , permettant de traiter les tumeurs ou affections cancéreuses par des cellules synthétisant des interleukines.

Le problème principal réside dans une possible survie et un possible développement dans l'organisme traité des cellules injectées codant pour les interleukines ou de virus dérivés de virus utilisés comme vecteurs.

Les demandeurs se sont donc attachés à la mise en oeuvre de compositions permettant de traiter de manière transitoire des organismes humains ou animaux par des substances biologiquement actives et ne présentant pas les inconvénients précités.

Ils ont mis en évidence de manière surprenante que l'on pouvait utiliser des lignées de cellules non syngéniques, et notamment des lignées de cellules allogéniques produisant des substances biologiquement actives pour traiter lesdits organismes . Ils ont ainsi montré en particulier que l'utilisation de cellules non syngéniques permettait une production transitoire d'interleukine dans lesdits organismes.

La présente invention a donc pour objet l'utilisation d'une composition destinée à traiter les organismes humains ou animaux, comprenant des cellules exprimant des gènes leur permettant de produire in vivo une ou plusieurs substances biologiquement actives caractérisée en ce que lesdites cellules présentent des caractéristiques génétiques les empêchant de se développer durablement dans l'organisme traité et les rendant susceptibles d'être éliminées artificiellement ou naturellement de l'organisme.

Ces substances biologiquement actives peuvent notamment être destinées à traiter de manière transitoire des organismes atteints par une tumeur ou une affection cancéreuse auquel cas lesdites substances peuvent être des interleukines. Les cellules seront alors choisies de manière à être éliminées après la disparition ou pendant la régression de la tumeur ou de l'affection cancéreuse.

Ces substances peuvent aussi être des molécules susceptibles d'induire une réaction immunitaire de type humoral ou cellulaire , tels que par exemple l'antigène HbS décrit dans le brevet français 8O.O9.O41 , un fragment de la glycoprotéine d'enveloppe du virus HIV ou tout autre antigène d'origine virale ou bactérienne, ou encore tout antigène normal ou mutant impliqué dans des pathologies, par exemple des antigènes spécifiques de tumeurs, ou impliqués dans des maladies autoimmunes,ou encore des anticorps ou des dérivés d'anticorps spécifiques . Outre leur utilisation dans le domaine de la vaccination ou de l'immunothérapie, ces cellules pourront aussi permettre de délivrer de façon transitoire d'autres substances actives, telles que des hormones , des facteurs de croissance ou leurs fragments.

Les cellules sont choisies afin que les organismes traités possèdent un système immunitaire permettant leur élimination . Ainsi , les cellules ne sont pas totalement syngéniques, elles sont au moins partiellement allogéniques. On entend par " cellule au moins partiellement allogénique ", une cellule qui se distingue de son organisme hôte par au moins un déterminant HLA.

Les cellules peuvent être aussi xénogéniques , bien que ce type de cellules puisse présenter l'inconvénient d'être rejetées plus rapidement et de produire une quantité plus faible de substances .

Mais il est aussi possible , de modifier des cellules allo- ou xénogéniques pour leur faire exprimer des antigènes caractéristiques des cellules humaines , par exemple des antigènes HLA de classe I ou de classe II et leur conférer des caractéristiques partiellement syngéniques de façon à stimuler transitoirement des réponses immunitaires caractéristiques de l'hôte .

Une lignée cellulaire particulièrement adaptée est la lignée VERO issue d'une espèce de singe . En effet , ces cellules présentent l'avantage d'avoir été étudiées et utilisées par de nombreuses équipes (voir notamment VERO cells; Origin, Properties and Biomedical Applications, Bunsiti Simizu et Toyozo Terasima, publié par le Département de Microbiologie de l'Ecole de Médecine de l'université de Chiba, Japon) . Ainsi , leur génétique est assez bien connue ce qui permet de réduire les risques d'infection dues à des virus ou à des rétrovirus endogènes . Ceci est particulièrement avantageux dans le cadre d'une thérapie humaine .

Les cellules de la composition mise en oeuvre selon l'invention peuvent aussi être sensibles à une drogue, ce qui permet de faciliter leur élimination par introduction de ladite drogue dans l'organisme . Une telle drogue peut être le gancyclovir auquel sont sensibles les cellules portant le gène de la thymidine kinase du virus de l'herpès.

Lesdits immunomodulateurs peuvent être notamment l'IL-2 , l'IL-4, le TNF ( Tumor Necrosis Factor), l'interféron gamma , et/ou le GM-CSF ( colony stimulating factor granulomonocytaire).

Les cellules peuvent produire seules ou en combinaison ces substances . De manière préférentielle, ces substances sont produites en quantités synergique . Avantageusement , une telle composition produit l'IL-2 et l'IL-4 en quantité synergique .

En outre, les cellules de cette composition peuvent porter un marqueur de coloration aisément identifiable . Il peut s'agir par exemple d'un gène codant pour la luciférase ou la β-galactosidase .

Afin de renforcer le caractère transitoire de l'expression des substances biologiquement actives et en particulier des immunomodulateurs dans les organismes traités, les gènes permettant aux cellules de produire ces substances peuvent être introduits dans les cellules par transfection et notamment par transfection sans sélection ultérieure de transfectant stable . On obtient un pool de cellules composé en majeure partie de cellules transfectées par l'ADN dans lesquelles celui-ci ne s'est pas intégré . De cette manière , les gènes et en particulier ceux codant pour les interleukines s'expriment mais leur ADN correspondant est éliminé rapidement au cours des cycles de division . Ceci permet de renforcer le caractère transitoire de l'expression des substances dans les organismes traités .

La transfection consiste en l'introduction d'ADN nu dans des cellules . Il s'agit d'une technique connue en soi et notamment décrite dans le Manuel Technique de Maniatis et al. (Molecular Cloning, A Laboratory Manual , Cold Spring Harbor Laboratory, 1982 ).

On pourra notamment utiliser la précipitation au phosphate de calcium , l'électroporation et occasionnellement des préparations de liposomes telles que la préparation commerciale Lipofectin Reagent (Bethesda Research Laboratories, Life Technologies , Inc.).

Les gènes portant les immunomodulateurs peuvent être obtenus par clonage , à partir d'ADN cellulaire notamment , ou par synthèse . On pourra notamment employer pour l'IL-2 et l'IL-4 les préparations d'ADN décrites dans Karasuyama et al. (Eur. J. Immunol. 1988,18:97).

De plus , les cellules utilisées dans la composition selon l'invention peuvent posséder des gènes leur permettant de produire des antigènes spécifiques de la tumeur ou de l'affection cancéreuse à traiter , afin d'accroître la réaction de l'organisme vis-à-vis de ces antigènes.

L'introduction de quantités supplémentaires d'antigène tumoral peut être aussi effectuée par addition aux cellules produisant les interleukines d'antigène synthétisé par voie chimique.

La composition mise en oeuvre selon l'invention peut comprendre plusieurs types cellulaires , chaque type cellulaire exprimant un immunomodulateur , ou être constituée d'un même type cellulaire produisant un ou plusieurs immunomodulateurs . L'invention concerne aussi tout traitement dans lequel une substance active peut être utilement délivrée in vivo de façon transitoire par des lignées de cellules manipulées au moins partiellement allo- ou xénogéniques .

Pour les raisons de simplicité de mise en oeuvre , il peut ainsi être intéressant de préparer des lignées cellulaires qui n'expriment qu'une seule interleukine . Toutefois , il peut aussi être avantageux que des mêmes cellules produisent des associations d'interleukine .

En outre , la présente invention concerne notamment l'utilisation des cellules ainsi définies pour la fabrication d'un médicament pour le traitement d'organismes humains ou animaux atteints par une tumeur ou une affection cancéreuse ainsi que des médicaments ou des vaccins contenant lesdites cellules.

On notera de plus , que de telles compositions sont destinées préférentiellement à être utilisées sous la forme d'injections localisées , mais peuvent aussi être utilisées de manière systémique . Des injections répétées peuvent être pratiquées, bien que l'on puisse soupçonner que leur efficacité pourrait décroître à mesure que le nombre d'injections est accrue, en raison d'un rejet accéléré par le système immunitaire .

Les quantités d'interleukines produites par ces cellules doivent être modulées en fonction du type de tumeur . A titre d'exemple , une expression de l'ordre de 4OOO unités internationales d'IL-2 / ml/1O⁶ cellules est efficace dans les cas décrits ci-dessous.

Néanmoins , pour des tumeurs à croissance lente, des doses plus faibles peuvent être efficaces.

Les présentes compositions sont préférentiellement utilisées dans le traitement de tumeurs solides mais peuvent être utilisées dans le traitement de tout autre type de tumeur ou affection cancéreuse .

La présente invention est illustrée sans pour autant être limitée par les exemples de mise en oeuvre suivants dans lesquels :

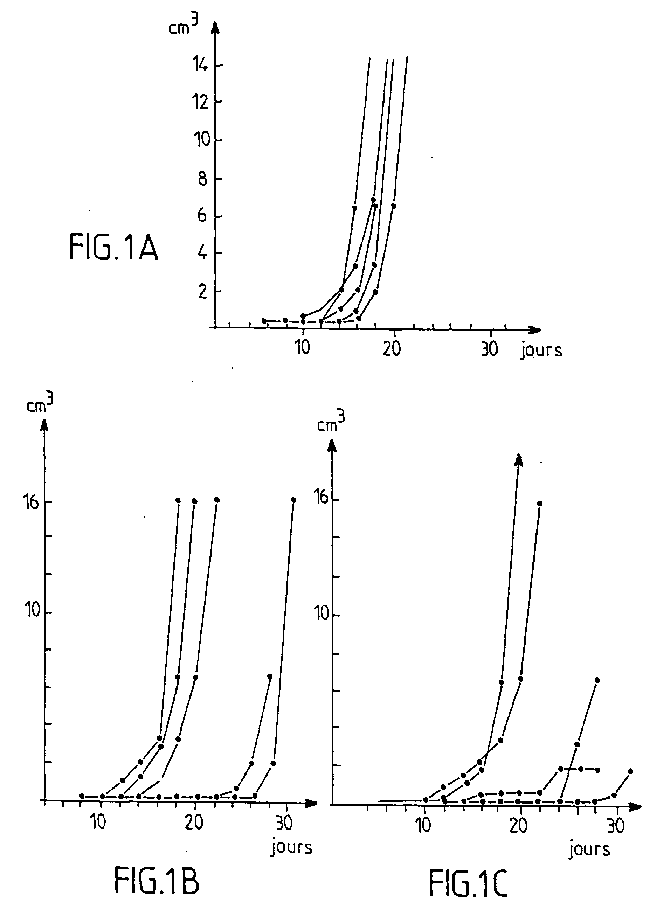

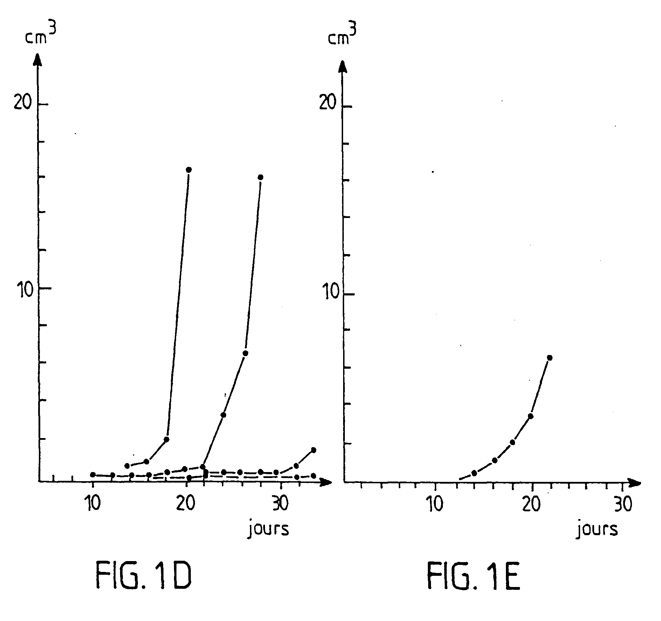

- les figures 1A à 1E sont des courbes illustrant l'évolution de la croissance de cellules tumorales en l'absence de cellules allogéniques (figure 1A ) en présence de cellules allogéniques ne synthétisant pas l'IL-2 ( figures 1B et 1C ) et en présence de cellules allogéniques produisant de l'interleukine-2 ( figures 1D et 1E ).

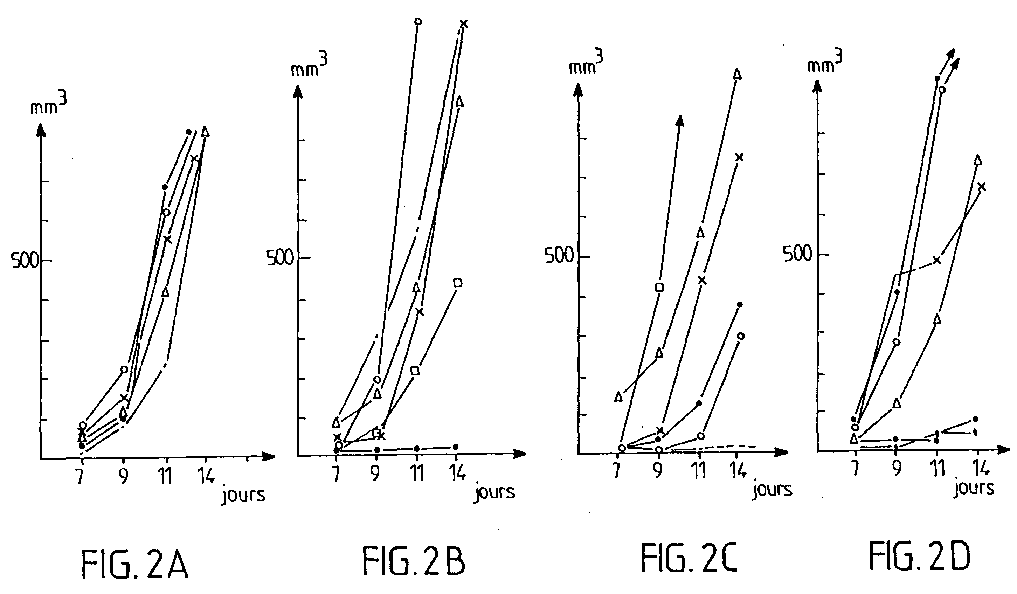

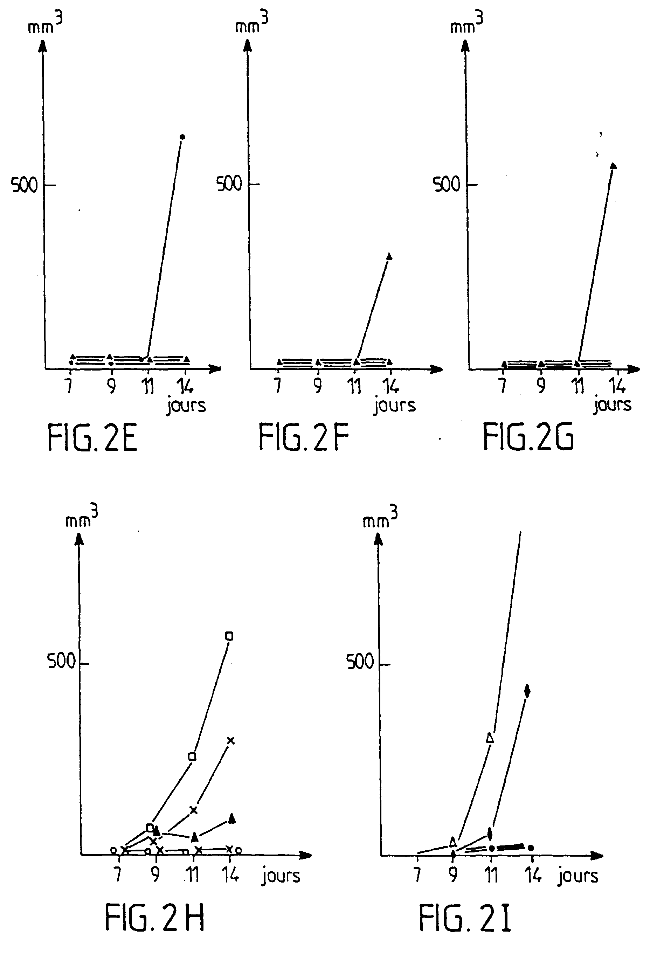

Les figures 2A à 2I montrent l'effet de cellules secrétant des interleukines sur le rejet de tumeurs de Lewis fraîchement implantées . La figure 2A est un témoin sans injection d'aucune cellule allogénique . Les figures 2B à 2D correspondent à l'injection de doses croissantes de cellules P815 non-transfectées . Les figures 2E à 2G correspondent à l'injection de cellules P815 produisant de l'IL-2 tandis que les figures 2H et 2I correspondent à l'injection de cellules P815 synthétisant respectivement l'IL-4 et une combinaison de cellules synthétisant l'IL-2 et l'IL-4 .

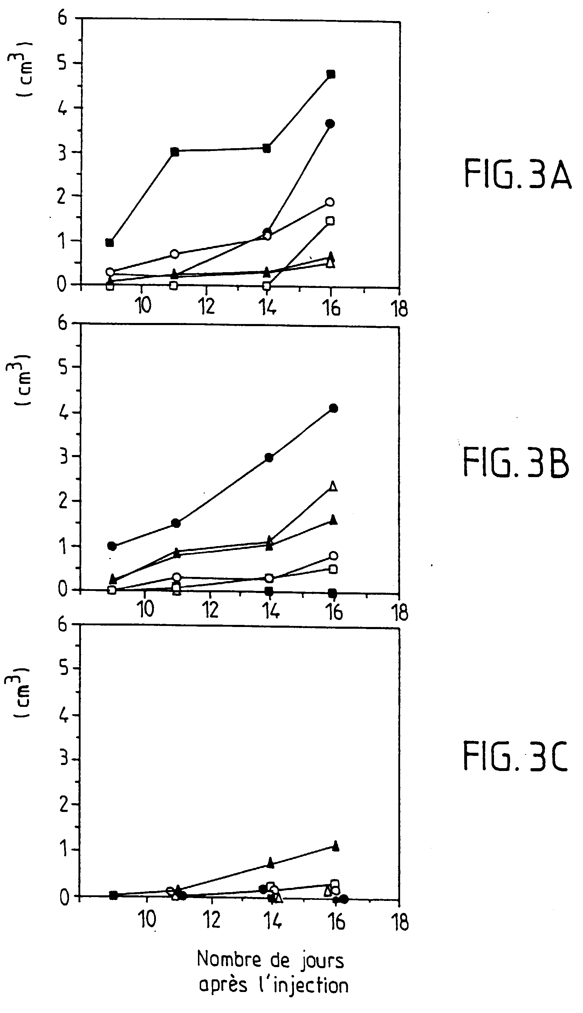

Les figures 3A à 3C sont relatives à la croissance de tumeurs de Lewis ( en ordonnées) dans des souris C57B1/6 en fonction du nombre de jours ( en abscisses), après incoulation par des cellules de tumeur de Lewis seules ( figure 3A) , des cellules de tumeurs de Lewis en présence de rMTC non-transfectées (figure 3B) et de rMTC transfectées par l'IL-2 (figure 3C).

### EXEMPLE 1 -

### Utilisation de cellules allogéniques produisant de l'IL-2 .

Des cellules L transformées d'haplotype H-2^{k} exprimant l'IL-2 et appelées LMI (IL-2 ) ont été isolées .

La lignée cellulaire LMI de souris exprime la molécule d'adhésion ICAM-l et est dérivée des cellules L (décrites dans le brevet français 80.O9.O41).

La lignée LMI a été décrite par Christian JAULIN ("Analyse structurale et fonctionnelle des antigènes d'histocompatibilité de classe I , Thèse de doctorat de l'Université Paris XI , 1991 ) .

On injecte à des souris DBA/2 d'haplotype H-2^{d} un mélange de 5.1O⁵ cellules P815 et de 1O⁶ ou de 5.1O⁶ cellules LMI ( IL-2 ) .

Les figures 1A à 1E illustrent les résultats obtenus.

Ces figures montrent que les cellules allogéniques LMI exprimant l'IL-2 confèrent une protection supérieure vis-à-vis de la croissance des cellules P815 co-injectées à celles conférées par des cellules LMI n'exprimant pas l'IL-2.

Les conditions opératoires des figures 1A à 1E sont les suivantes:
Figure 1A 5.1O⁵ cellules P815,
Figure 1B 5.1O⁵ P815 + 1O⁶ cellules L ,
Figure 1C 5.1O⁵ cellules P815 + 5.1O⁶ cellules
Figure 1D 5.1O⁵ cellules P815 + .1O⁶ cellules LMI (IL-2) ,
Figure 1E 5.1O⁵ cellules P815 + 5.1O⁶ cellules LMI (IL-2 ) .

Sur les cinq souris correspondant à la figure 1D deux sont totalement protégées , une développe une tumeur très tardivement tandis que les deux dernières font des tumeurs rapidement .

Dans les conditions opératoires de la figure 1E, une seule souris fait une tumeur rapidement , les quatre autres étant totalement protégées .

Sur les figures 1A à 1E , les chiffres indiqués en abscisse correspondent au nombre de jour après injection, tandis que l'ordonnée indique le volume de la tumeur en cm³.

### EXEMPLE 2-

### Effet de cellules allogéniques produisant des Interleukines à l'encontre de tumeurs de Lewis .

5.1O⁵ cellules isolées de tumeurs de Lewis fraîchement implantés ( haplotype H-2b ) mélangées avec différentes quantités de P815 ( IL-2 ) ou de P815 ( IL-4 ) sont injectées à des souris C57B1/6 (haplotype H-2^{b}).

Les cellules P815 sont d'haplotype H-2^{d}.

Les figures 2A à 2I sont réalisées dans les conditions suivantes :
Figure 2A 5.1O⁵ cellules de Lewis ,
Figure 2B 5.1O5 cellules de Lewis + 5.1O⁵ cellules P815 ,
Figure 2C 5.1O⁵ cellules de Lewis + .1O⁶ cellules P815 ,
Figure 2D 5.1O⁵ cellules de Lewis + 2.1O⁶ P815,
Figure 2E 5 1O⁵ cellules de Lewis + 5.1O⁵ P815 (IL-2) ,
Figure 2F 5.1O⁵ cellules de Lewis + .1O⁶ P815 (IL-2),
Figure 2G 5.1O⁵ cellules de Lewis + 2.1O⁶ P815 (IL-2),
Figure 2H 5.1O⁵ cellules de Lewis + 1O⁶ P815 (IL-4),
Figure 2I 5.1O⁵ cellules de Lewis + 5.1O⁵ P815 (IL-2) + 5.1O⁵ P815 (IL-4).

Sur ces figures , l'abscisse indique que le nombre de jour après le traitement et l'ordonnée indique le volume des tumeurs exprimé en mm³ .

Ces résultats montrent donc que les cellules P815 (IL-2) confèrent une haute et reproductible protection tandis que les cellules P815 non transfectées ne confèrent pas ce type de protection .

Les cellules P815 IL-4 ( figure 2H) confèrent aussi une protection bien que plus faible que celle conférée par les P815 ( IL-2 ) .

On observe par contre pas de synergie entre l'effet des P815 ( IL-2 ) et l'effet des P815 ( IL-4) (figure 2I ) , mais au contraire un effet plutôt antagoniste .

### EXEMPLE 3.

### Rejet des tumeurs de Lewis dans des souris C57B1/6 induit par des cellules tumorales secrétant de l'Interleukine-2.

Le carcinome thyroidien médulaire de rat (rMTC) est un néoplasme spontané dérivé de cellules C intra-thyroïdales produisant de la calcitonine . La lignée cellulaire spécifique obtenue à partir de ces cellules (rMTC 6.23) a été décrite ( Zeytinoglu et al. (1980) Endocrinology 107:5O9).

La capacité de cette souche qui secrète des quantités importantes d'Interleukine-2 ( 5OOO U/ml/10⁶ cellules/24 heures ) à induire une protection immunitaire anti-tumorale dans un hôte xénogénique a été testée . Des souris C57B1/6 ont été inoculées avec soit 2,5 x 10⁵ cellules tumorales de Lewis seules soit avec ces cellules on combinaison avec 10⁶ cellules de rats rMTC ( IL-2) .

Les cellules xénogéniques produisant de l'Interleukine-2 induisent une protection significative comme le montre la figure 3.

La figure 3A concerne l'inoculation sous-cutanée de six souris C57B1/6 avec 2,5.10⁶ cellules tumorales de Lewis seules .

Les figures 3B et 3C correspondent respectivement à la combinaison de 2,5 10⁵ cellules de Lewis avec 10⁶ cellules rMTC non transfectées ( figure 3B) ou avec 10⁶ cellules rMTC transfectées par l'Interleukine-2 ( figure 3C).

Tous les animaux sans tumeurs au 6ème jour ne développent pas de tumeur après 6O jours.

On notera néanmoins , malgré la mise on évidence d'une protection significative, qu'il est nécessaire dans les mêmes conditions d'ajouter quatre foix plus de cellules xénogéniques produisant de l'Interleukine-2 que de cellules allogéniques .

### EXEMPLE 4:

### Influence de la présence de cellules NK-1.1 sur le rejet des tumeurs.

L'effet de l'élimination sélective in vivo de cellules Natural Killer (NK) sur le rejet de tumeurs dans des souris C57B1/6 co-inoculées avec des cellules de tumeurs de Lewis et des cellules allogéniques P815 ( IL-2) a été testée .

L'élimination in vivo par les anticorps a été effectuée par injection intra-péritonéale de 100 µg d'anticorps monoclonal spécifique de NK purifié, durant 3 jours, en commençant 1 jour avant l'injection des cellules tumorales .

L'efficacité de l'élimination des cellules NK a été évaluée à l'aide d'un test de relarguage du chrome 51 en utilisant des cellules cibles YAC1 ( Kiessling et al. 1975, Eur. J. Immunol., 5:112 ) , comme décrit par Koo et al. ( 1986, J. Immunol., 137:3742).

On a vérifié que le traitement était efficace sur les activités Natural Killer endogènes et induites par du Poly-IC de la lignée cellulaire YAC-1 dans la rate d'animaux traités .

Les résultats sont résumés sur le tableau I ci-après .

Par comparaison avec des animaux non traités , les tumeurs se développent de manière plus rapide dans des souris auxquelles on a injecté des anticorps anti-NK. De plus , l'évaluation de la protection tumorale montre que les souris dont on a éliminé les cellules NK-1.1 par un traitement d'anticorps sont incapables d'inhiber la croissance tumorale des cellules de Lewis (tableau I).

Il est néanmoins à noter que l'inoculation de cellules allogéniques secrétant des lymphokines à ces animaux traités entraîne un retard quant à l'apparition des tumeurs et une baisse de leur volume moyen .

### EXEMPLE 5:

### Etude du rejet de cellules tumorales de Lewis par des souris ayant au préalable rejeté des cellules tumorales du même type .

Trois groupes de souris C57B1/6 sur lesquelles ont déjà été effectuées des expériences de rejet de cellules tumorales de Lewis co-inoculées avec des cellules P815 (IL-2) ont été testées six semaines après le premier rejet avec des cellules tumorales de Lewis ( 5x10⁵).

Aucune des souris testées n'a survécu à ces injections. On a cependant remarqué que la croissance tumorale a été légèrement retardée par rapport à la croissance chez des animaux témoins n'ayant pas été au préalable traités par des cellules de tumeur de Lewis et des cellules P815 (IL-2).

En conclusion , l'ensemble de ces résultats a permis de montrer :
- que la thérapie par des cellules exprimant des gènes d'interleukines est possible pour des tumeurs spontanées, et non seulement pour des tumeurs induites chimiquement connue décrit dans l'art antérieur ,
- l'utilisation de cellules allogéniques est possible et permet le rejet des tumeurs ,
- la délivrance par expression transitoire d'une substance biologiquement active est possible ,
- on peut utiliser des cellules allogéniques ou partiellement allogéniques comme porteur d'un antigène étranger à l'hôte susceptible d'induire une réaction immunitaire de type humorale ou cellulaire ,
- les cellules " Natural Killer " (NK) interviennent dans la protection induite par l'IL-2 exprimée par les cellules allogéniques .

**TABLEAU I**

| Croissance des tumeurs de Lewis dans des souris C57B1/6 traitées avec un anticorps monoclonal anti-NK 1.1* | | | | | |
|---|---|---|---|---|---|
| Traitement in vivo ** | Cellules injectées | | Volume moyen de la tumeur (cm3) | | Fraction d,animaux protégés • |
| | tumeur parentale | cellules allogéniques (5.10⁵) | D₁⁺ | D₂⁺⁺ | |
| - | Lewis | - | 0.11 ± O.O7 | 1.76 + O.42 | O/5 |
| Anti-NK 1.1 | Lewis | - | O.25 ± 0.18 | 2.2 + 0.60 | O/5 |
| Anti-NK 1.1 | Lewis | P815 (5.10⁵) | 0.42 ± 0.20 | 2.30 ± O.58 | O/5 |
| Anti-NK 1.1 | Lewis | P815-(IL2)(5.10⁵) | 0.12 + 0.15 | 2.0 ± 0.7 | O/5 |
| Anti-NK 1.1 | Lewis | P815-(IL4)(5.10⁵) | 0.14 ± 0.22 | 2.8 ± 1.7 | O/5 |
| Anti-NK 1.1 | Lewis | P815-(IL2)(5.10⁵) | O | 1.3 ± O.4 | O/5 |
| | | P815-(IL4)(5.10⁵) | | | |

| | | | | | |
|---|---|---|---|---|---|
| • Le pourcentage de relarguage spécifique du chrome ⁵¹Cr après incubation des cellules cibles durant 4, heures à 37°C est négligeable pour les animaux traités, tandis que dans les cellules de rate induites par le Poly-IC , il est de 54 et 23% respectivement au bout d'un jour et de 8 jours après un traitement in vitro avec un rapport cellules-cibles : cellules effectrices de 1/100. | | | | | |
| ** Le traitement a été effectué à l'aide d'anticorps décrit par Koo et Peppard ((1984) Hybridoma 3:301). | | | | | |
| +D1 : mesure du volume tumoral 9 jours après l'inoculation des souris C57B1/6. | | | | | |
| +D2 : mesure du volume tumoral 16 jours après l'inoculation des souris C57B1/6. | | | | | |
| •: La fraction des animaux protégés est mesurée est la fraction d'animaux sans tumeur détectable 3O jours après l'inoculation. | | | | | |

## Revendications

1. Utilisation d'une composition comprenant des cellules au moins partiellement allogéniques ou xénogéniques vis-à-vis d'un organisme cible à traiter, exprimant des gènes leur permettant de produire i*n vivo* une ou plusieurs substances biologiquement actives, pour la préparation d'un médicament destiné au traitement transitoire des organismes humains ou animaux.

2. Utilisation selon la revendication 1 caractérisée en ce que ledit traitement est un traitement d'une affection cancéreuse ou d'une tumeur

3. Utilisation selon la revendication 1 caractérisée en ce que ledit traitement est un traitement destiné à induire une réaction immunitaire de type tumorale ou cellulaire.

4. Utilisation selon l'une quelconque des revendications 1 à 3 caractérisée en ce que lesdites substances biologiquement actives sont des immunomodulateurs.

5. Utilisation selon la revendication 4 caractérisée en ce que lesdits immunomodulateurs sont l'IL-2, l'IL-4, le TNF, l'interféron gamma, et/ou le GM-CSF.

6. Utilisation selon la revendication 3 caractérisée en ce que lesdites substances biologiquement actives sont sélectionnées parmi l'antigène HbS, un fragment de la glycoprotéine d'enveloppe du virus HIV, un fragment d'un antigène d'origine virale ou bactérienne, un antigène spécifique de tumeur, un antigène impliqué dans une maladie autoimmune, un anticorps ou d'un dérivé d'anticorps.

7. Utilisation selon la revendication 1 caractérisée en ce que ladite substance active est sélectionnée parmi les hormones, les facteurs de croissance ou leurs fragments.

8. Utilisation selon l'une quelconque des revendications 1 à 7 caractérisée en ce que lesdites cellules sont sensibles à une drogue favorisant leur élimination de l'organisme.

9. Utilisation selon la revendication 8 caractérisée en ce que ladite drogue est le gancyclovir, auquel cas les cellules portent le gène de la thymidine-kinase du virus de l'herpès.

10. Utilisation selon la revendication 4 caractérisée en ce que lesdites cellules produisent en quantités synergiques les immunomodulateurs.

11. Utilisation selon la revendication 10 caractérisé en ce que lesdites cellules produisent l'IL-2 et l'IL-4 en quantités synergiques.

12. Utilisation selon l'une quelconque des revendications 1 à 11 caractérisée en ce que lesdites cellules portent un marqueur de coloration.

13. Utilisation selon l'une quelconque des revendications 1 à 12 caractérisée en ce que les gènes ont été introduits dans les cellules par transfection.

14. Utilisation selon la revendication 2 ou 4 caractérisée en ce que les cellules possèdent en plus des gènes leur permettant de produire des antigènes spécifiques de la tumeur ou de l'affection cancéreuse à traiter.

15. Utilisation selon l'une quelconque des revendications 1 à 14 caractérisée en ce que ladite composition est composée de plusieurs types cellulaires produisant chacun un ou plusieurs immunomodulateurs et/ou antigène spécifique de la tumeur à traiter.

## Claims

1. Use of a composition comprising cells which are at least partially allogenic or xenogenic for the target organism to be treated, wherein said cells express genes enabling them to produce *in vivo* one or several biologically active substances, for the manufacture of a medicament for the transitory treatment of human or animal organisms.

2. Use according to claim 1, wherein said treatment consists of a treatment of a cancer or a tumour.

3. Use according to claim 1, wherein said treatment consists of a treatment for inducing a humoral or cellular immune reaction.

4. Use according to any one of claims 1 to 3, wherein said biologically active substances are immunomodulators

5. Use according to claim 4, wherein said immunomodulators are IL-2, TNF, gamma interferon and/or GM-CSF.

6. Use according to claim 3, wherein said biologically active substances are selected among the HbS antigen, a fragment of the envelope glycoprotein from the HIV virus, a fragment of an antigen of viral or bacterial origin, a tumour-specific antigen, an antigen involved in an autoimmune disease, an antibody or an antibody derivative.

7. Use according to claim 1, wherein said active substance is selected among homones, growth factors or their fragments.

8. Use acording to any one of claims 1 to 7, wherein said cells are sensitive to a drug promoting their elimination from the organism.

9. Use according to claim 8, wherein said drug is gancyclovir, in which case said cells carry the thymidine kinase gene form the Herpes virus.

10. Use according to claim 4, wherein said cells produce the immunomodulators in synergistic quantities.

11. Use according to claim 10, wherein said cells produce IL-2 and IL-4 in synergistic quantities.

12. Use according to any one of claims 1 to 11, wherein said cells carry a dye marker.

13. Use according to any one of claims 1 to 12, wherein the genes have been introduced in the cells by transfection.

14. Use according to any one of claims 2 or 4, wherein the cells additionally possess genes enabling them to produce specific antigens of the tumour or of the cancer to be treated.

15. Use according to any one of claims 1 to 14, wherein said composition is composed of several cell types each producing one or several immunomodulators and/or antigen specific for the tumor to be treated.

## Patentansprüche

1. Verwendung einer Zusammensetzung, die Zellen umfasst, welche gegenüber einem zu behandelnden Zielorganismus wenigstens zum Teil allogen oder xenogen sind und Gene exprimieren, die es ihnen ermöglichen, eine oder mehrere biologisch aktive Substanzen in vivo zu produzieren, für die Herstellung eines Medikaments zur vorläufigen Behandlung menschlicher oder tierischer Organismen.

2. Verwendung gemäß Anspruch 1, dadurch gekennzeichnet, dass es sich bei der Behandlung um die Behandlung einer Krebserkrankung oder eines Tumors handelt.

3. Verwendung gemäß Anspruch 1, dadurch gekennzeichnet, dass es sich bei der Behandlung um eine Behandlung zur Induktion einer Immunreaktion des humoralen oder zellulären Typs handelt.

4. Verwendung gemäß einem der Ansprüche 1 bis 3, dadurch gekennzeichnet, dass es sich bei den biologisch aktiven Substanzen um Immunmodulatoren handelt.

5. Verwendung gemäß Anspruch 4, dadurch gekennzeichnet, dass es sich bei den Immunmodulatoren um IL-2, IL-4, TNF, Interferon γ und/oder GM-CSF handelt.

6. Verwendung gemäß Anspruch 3, dadurch gekennzeichnet, dass die biologisch aktiven Substanzen ausgewählt sind aus dem HBs-Antigen, einem Fragment des Hüllglycoproteins des Virus HIV, einem Fragment eines Antigens viralen oder bakteriellen Ursprungs, einem spezifischen Tumorantigen, einem Antigen, das an einer Autoimmunerkrankung beteiligt ist, einem Antikörper oder einem Antikörperderivat.

7. Verwendung gemäß Anspruch 1, dadurch gekennzeichnet, dass die aktive Substanz ausgewählt ist aus Hormonen, Wachstumsfaktoren oder ihren Fragmenten.

8. Verwendung gemäß einem der Ansprüche 1 bis 7, dadurch gekennzeichnet, dass die Zellen gegenüber einem Wirkstoff empfindlich sind, der ihre Entfernung aus dem Organismus begünstigt.

9. Verwendung gemäß Anspruch 8, dadurch gekennzeichnet, dass es sich bei dem Wirkstoff um Gancyclovir handelt und die Zellen das Gen der Thymidin-Kinase des Herpesvirus tragen.

10. Verwendung gemäß Anspruch 4, dadurch gekennzeichnet, dass die Zellen die Immunmodulatoren in synergistischen Mengen produzieren.

11. Verwendung gemäß Anspruch 10, dadurch gekennzeichnet, dass die Zellen IL-2 und IL-4 in synergistischen Mengen produzieren.

12. Verwendung gemäß einem der Ansprüche 1 bis 11, dadurch gekennzeichnet, dass die Zellen einen Farbmarker tragen.

13. Verwendung gemäß einem der Ansprüche 1 bis 12, dadurch gekennzeichnet, dass die Gene durch Transfektion in die Zellen eingeschleust worden sind.

14. Verwendung gemäß Anspruch 2 oder 4, dadurch gekennzeichnet, dass die Zellen außerdem Gene besitzen, die es ihnen ermöglichen, spezifische Antigene des zu behandelnden Tumors oder der zu behandelnden Krebserkrankung zu produzieren.

15. Verwendung gemäß einem der Ansprüche 1 bis 14, dadurch gekennzeichnet, dass die Zusammensetzung aus mehreren Zelltypen besteht, von denen jeder einen oder mehrere Immunmodulatoren und/oder spezifische Antigene des zu behandelnden Tumors produziert.
